# EUROPEAN PATENT SPECIFICATION

(11) **EP 2 328 484 B1**
(45) Date of publication and mention of the grant of the patent: **01.07.2015**
(21) Application number: 09759064.0
(22) Date of filing: 27.05.2009
(51) Int. Cl.: A61B 17/17, A61B 17/72, A61M 25/00

(54) **CATHETER NAIL TARGETING GUIDE**
KATHETERNAGEL-TARGETING-FÜHRUNG
GUIDE DE CIBLAGE POUR CLOU DE CATHÉTER

(30) Priority: 03.06.2008 US 132310
(43) Date of publication of application: 08.06.2011
(73) Proprietor: Zimmer, Inc., Warsaw, IN 46580 (US)
(72) Inventor: WACK, Michael, A., Warsaw IN 46580 (US); MUTCHLER, Austin, W., Warsaw IN 46580 (US)
(74) Representative: Mays, Julie
(86) International application number: PCT/US2009/045285
(87) International publication number: WO 2009/148903

(56) References cited:
- FR-A- 2 587 196
- US-A- 4 865 025
- US-A- 5 374 271
- US-B2- 7 311 710

## Description

### BACKGROUND

### 1. Field of the Invention

The present invention relates to an instrumentation guide usable in orthopedic surgery. In particular, the present invention relates to an instrumentation guide that may be used with intramedullary nails or rods.

### 2. Description of the Related Art

Intramedullary nails are often used by orthopedic surgeons to secure a fracture in a long bone, such as a femur, for example. Once the intramedullary nail has been inserted into the intramedullary canal of the fractured bone, screws may be inserted through the bone and nail. In general, the screws are arranged at an angle relative to the longitudinal axis of the nail. An instrumentation guide, or targeting device, may be typically used to align the screws with receiving apertures in the nail. An example of a known instrumentation guide utilized to align screws in a typical procedure is disclosed in U.S. Patent No. 7,144,399, assigned to the assignee of the present invention. An alternative guide is described in US Patent No. 5,374,271.

The instrumentation guide is generally secured to an end of the nail in order to fix the position of the nail with respect to the body of the guide. The body of the guide may then by used to align the drill and screws with the receiving apertures formed in the nail.

### SUMMARY

The present invention relates to a combination of an instrumentation guide or targeting guide and an intramedullary nail. The guide includes a guide body having a handle portion and a barrel portion. The intramedullary nail is fixedly securable to the barrel portion via a locking bolt. The barrel portion includes at least one canal configured to receive a catheter, the canal aligned with a corresponding canal in the intramedullary nail. In use, a catheter may be inserted over a guide wire and through both the canal in the barrel portion of the guide body and through the corresponding canal in the intramedullary nail prior to insertion of the intramedullary nail into the intramedullary canal of a long bone. In this manner, the placement of the catheter for delivery of medications within in the intramedullary canal of the long bone is facilitated.

In one embodiment, the barrel portion includes two canals each alignable with corresponding canals in the intramedullary nail, and each configured to receive a catheter.

In one form thereof, the present invention provides a guide for use with an intramedullary nail, said nail having at least one first canal, said guide comprising a guide body including a handle portion and barrel portion, said barrel portion engageable with the intramedullary nail and including at least one second canal, said second canal alignable with said first canal along a common axis.

In another form thereof, the present invention provides, in combination, a targeting guide including a handle portion and a barrel portion, said barrel portion including at least one first canal and an intramedullary nail secured to said barrel portion, said intramedullary nail including at least one second canal said intramedullary nail aligned with said first canal.

Also described is a method for positioning a catheter within a long bone, the method including the steps of securing an intramedullary nail to a barrel portion of a targeting guide; aligning at least one first canal in the barrel portion with at least one second canal in the intramedullary nail; inserting a catheter through the aligned first and second canals; inserting the intramedullary nail into the intramedullary canal of a long bone; securing the intramedullary nail with respect to the long bone; detaching the targeting guide from the intramedullary nail; and separating the targeting guide from the catheter.

### BRIEF DESCRIPTION OF THE DRAWINGS

The above-mentioned and other features of this invention, and the manner of attaining them, will become more apparent and the invention itself will be better understood by reference to the following description of an embodiment of the invention taken in conjunction with the accompanying drawings, wherein:
Fig. 1A is a perspective view of a targeting guide representing an embodiment of the invention;
Fig. 1B is a sectional view taken along line 1B-1B of Fig. 1A;
Fig. 2 is a lower perspective view of the embodiment of the invention depicted in Fig. 1;
Fig. 3 is an exploded perspective view of the embodiment of the invention depicted in Fig. 1;
Fig. 4 is a perspective view of the embodiment of the invention depicted in Fig. 1 inserted into a bone;
Fig. 5 is a perspective view of the embodiment of the invention depicted in Fig. 4 with a bone screw inserted into the nail; and
Fig. 6 is a perspective view of the embodiment of the invention depicted in Fig. 5 separated from the nail.

Corresponding reference characters indicate corresponding parts throughout the several views. The exemplification set out herein illustrates an embodiment of the invention and such exemplification is not to be construed as limiting the scope of the invention in any manner.

### DETAILED DESCRIPTION

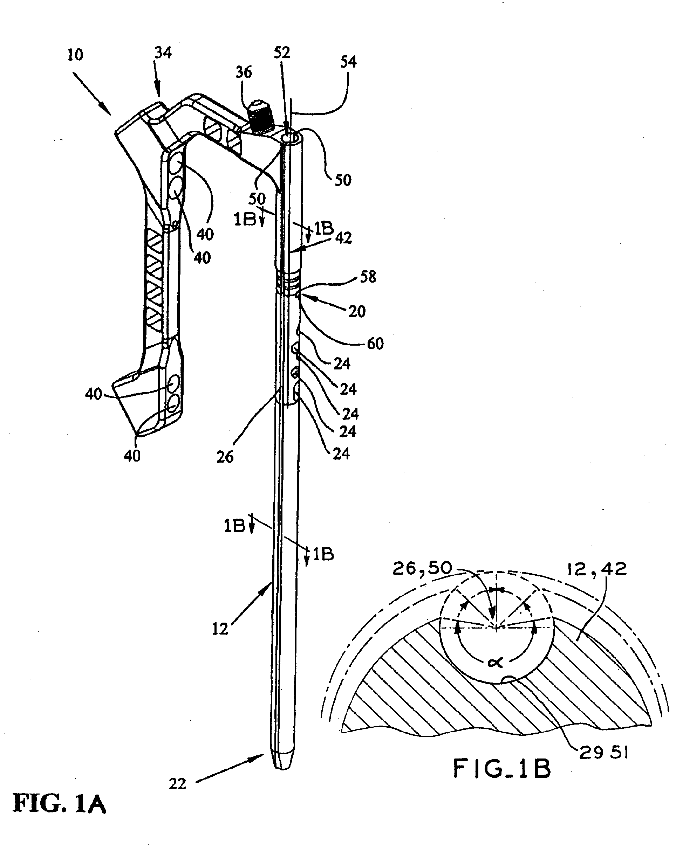

Fig. 1A depicts an embodiment of an instrumentation or targeting guide, generally indicated by numeral 10. In the present embodiment, targeting guide 10 is configured for use with an intramedullary nail, indicated by numeral 12.

With reference now to Figs. 1A through 3, nail 12 includes a proximal end, generally indicated by numeral 20, and a distal end, generally indicated by numeral 22. In the depicted embodiment, nail 12 includes a plurality of screw receiving apertures 24 and a pair of catheter canals 26 which extend longitudinally along opposite sides of nail 12. Apertures 24 are configured to receive bone screws in a conventional manner, and the catheter canals 26 are configured to receive catheters 27. Catheters 27 may be any suitable catheter known in the art.

Referring still to Fig. 1A, targeting guide 10 includes a handle portion 34 and a threaded member 36. Handle portion 34 may be formed from any suitable biocompatible material, which may be a radiolucent material such as a composite of polyetheretherketone (PEEK) and carbon fibers.

Handle portion 34 may be formed in any suitable manner, such as via a two-piece construction including two molded portions, or handle portion 34 may be molded or machined as a single piece construction.

In the depicted embodiment, handle portion 34 includes a plurality of apertures, each indicated by numeral 40. The apertures 40 are sized and configured to guide a drill and bone screw in a conventional manner during a standard surgical procedure. Handle portion 34 further includes a barrel or barrel portion 42. Barrel portion 42 is configured to receive a locking bolt 62 and mate with the nail 12 in a conventional manner.

Threaded member 36 may be molded into the handle portion 34, or alternatively, threaded member 36 may be located within a receiving area (not shown) formed in handle portion 34 and retained therein in any suitable manner. Threaded member 36 is configured to facilitate the delivery of insertion forces. For example, during insertion of nail 12, a cap (not shown) may be attached to the threaded member 36 in order to allow a surgeon to utilize a suitable instrument, such as a hammer or impactor, in inserting the nail into the bone.

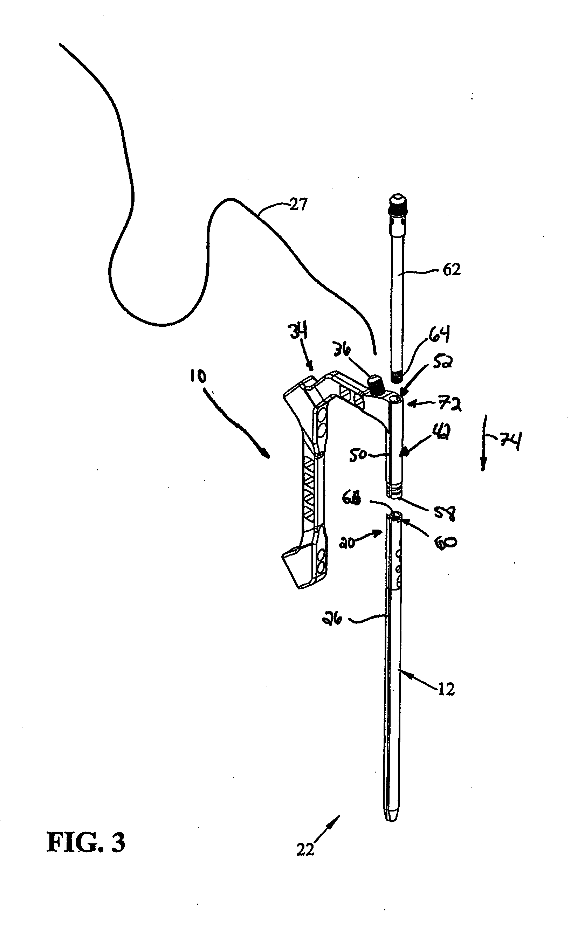

With reference still to Fig. 1A, barrel 42 includes a pair of canals, each indicated by numeral 50. The canals 50 are sized and configured to receive a catheter, and are correspondingly sized with respect to, and alignable with, canals 26 in intramedullary nail 12. Referring to Figs. 1A and 1 B, canals 26 and 50 are each shaped to contain a catheter 27 (Fig. 3) that has a generally circular cross-section. As shown in Fig. 1A, canals 26 and 50 are substantially circular, and include an inner surface 29, 51 that extends through, or encompasses, an angle α of at least 180° in order to retain catheter 27 therein. Also, the inner surfaces 29, 51 of canals 26, 50 may extend through, or encompass, an angle α of 360°, in which canals 26, 50 are formed as completely enclosed bores. As schematically represented by dashed lines in Fig. 1 B, the angle α encompassed by the inner surfaces 29, 51 of canals 26, 50may be between 180° and 360° and, in further embodiments, may be as little as 185°, 195°, or 200°, or as great as 270°, 300°, or 350°, for example and, in further embodiments, may be between 250° and 290°. Still further, canals 26 and 50 may have the same shape, or different shapes.

Barrel 42 further includes a bore 52 extending substantially along its longitudinal axis 54. In the depicted embodiment, bore 52 may be sized and configured to receive any suitable locking bolt known in the art.

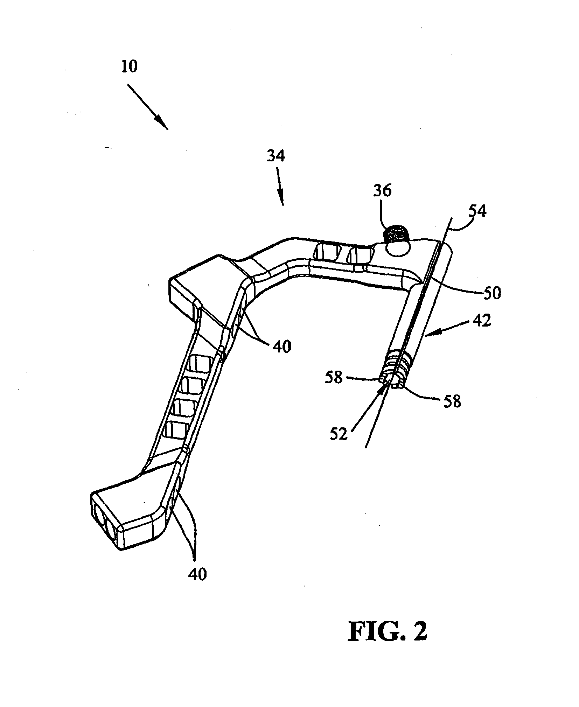

Referring now to Figs. 2 and 3, barrel 42 also includes a pair of extensions 58. The extensions 58 are configured to mate with the end of nail 12. Specifically, the extensions 58 extend into the receiving areas 60 present within the proximal end 20 of nail 12. It should be noted that the extensions 58 and receiving areas 60 may be replaced with any suitable mechanism for allowing barrel 42 to mate with nail 12.

Referring still to Figs. 1-3, nail 12 may be connected to targeting guide 10 in a suitable manner, for example. A locking bolt 62, shown in Fig. 3, may be utilized to affix the targeting guide 10 to nail 12 in a conventional manner. For example, nail 12 may include a portion configured to receive threaded portions of the locking bolt.

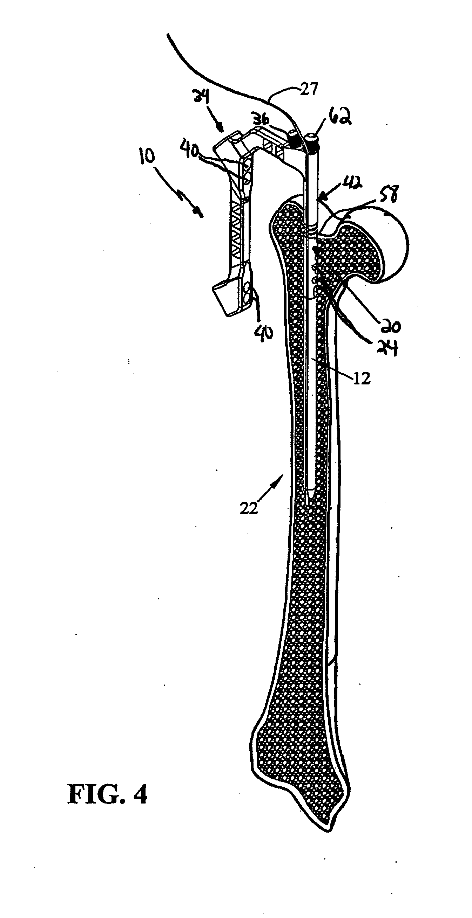

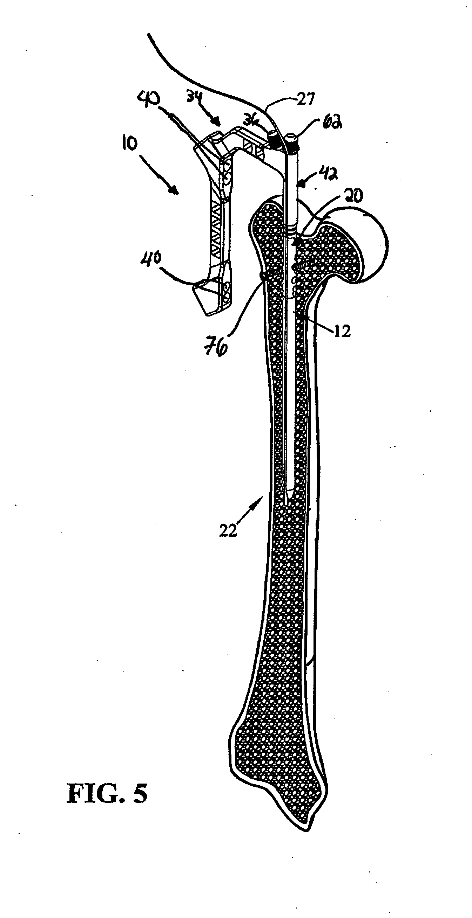

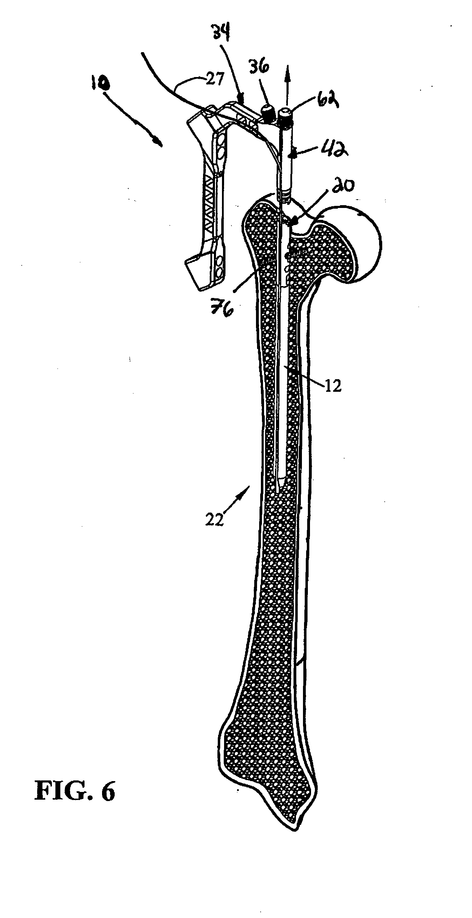

With reference now to Figs. 1 through 6, a method of employing the targeting guide 10 will now be described. In describing the method, it should be understood that the intramedullary canal of bone may be prepared to receive the nail 12 in any suitable manner. In order to insert the nail 12 into the intramedullary canal, the nail 12 is connected to the guide 10. Specifically, the extensions 58 of guide 10 are located within the receiving areas 60 of the nail 12. A suitable locking bolt 62 may be inserted into the bore 52 of barrel 42. The threads 64 of the locking bolt 62 are configured to engage the threads 66 of the nail 12, in a suitable manner. The locking bolt 62 may then be rotated in order to lock the nail 12 to the guide 10. It should be noted that the canals 50 of barrel 42 align with canals 26 of the nail 12.

Once the nail 12 has been connected to guide 10, catheters 27 may be inserted into the canals 50 of the guide 10. The catheters 27 may be any suitable type of catheter known in the art. The catheters 27 may be threaded into the canals 50 from the end, generally indicated by numeral 72. The catheters 27 may then be inserted in the direction of arrow 74. Once the catheters 27 have been inserted through the canals 50 in their entirety, the catheters 27 may be inserted into the canals 26 of the nails 12. The catheters 27 may continue to be inserted in the direction of arrow 74 until the catheters 27 traverse the canals 26 in their entirety.

Once the nail 12 has been connected to the guide 10, the nail 12 may be inserted into the intramedullary canal of the bone in a suitable manner. Once the nail 12 has been located in the intramedullary canal, the bone screws 76 may be inserted into the apertures 40 of the handle portion 34 and inserted into the bone and the screw receiving apertures 24 of nail 12 in order to affix the nail 12 to bone.

The catheters 27 may include a wire (not shown) that is included in the catheters 27 in order to prevent the catheters 27 from collapsing during surgery. Once the nail 12 has been inserted into the intramedullary canal of bone, the wire may be removed from the catheters 27. The guide 10 may be disconnected from the nail 12 by rotating the locking bolt 62 in a suitable manner. As the targeting guide 10 is disconnected from nail 12, the remainder of the catheters 27 pass through canals 50 of the barrel 42. The catheters 27 may then be used to allow adhesives or drugs to facilitate the healing of the bone.

While this invention has been described as having exemplary designs, the present invention may be further modified. This application is therefore intended to cover any variations, uses, or adaptations of the invention using its general principles. Further, this application is intended to cover such departures from the present disclosure as come within known or customary practice in the art to which this invention pertains.

## Claims

1. In combination:
a targeting guide (10) including a handle portion (34) and a barrel portion (42), said barrel portion (42) including bore (52) extending substantially along its longitudinal axis (54) and at least one first canal (50); and
an intramedullary nail (12) secured to said barrel portion (42), said intramedullary nail (12) including at least one second canal (26) aligned with said first canal (50) said canals (26, 50) configured to receive a catheter (27).

2. The combination of claim 1, wherein said barrel portion (42) includes a pair of said first canals (50) and said intramedullary nail (12) includes a pair of said second canals (26), said first (50) and second canals (26) respectively aligned with one another.

3. The combination of claim 2, wherein at least one of said first (50) and second (26) canals includes an inner surface (29, 51) that extends through an angle of at least 180°.

4. The combination of claim 1, wherein said first (50) and second (26) canals are aligned along a common first axis.

5. The combination of claim 1, wherein said barrel portion (42) and said intramedullary nail (12) are aligned along a common second axis, said second axis parallel to said first axis.

6. The combination of claim 1, wherein said barrel portion (42) further comprises means for fixedly engaging said intramedullary nail (12).

7. The combination of claim 1, wherein the catheter (27) may be inserted through the aligned canals (26, 50).

8. The combination of claim 1, wherein the combination further comprises a catheter (27).

9. The combination of claim 8, wherein the catheter (27) is received in the canal (26) of the barrel portion (42) and the canal (50) in the intramedullary nail (12) prior to insertion of intramedullary nail (12) into an intramedullary canal of a long bone so that placement of the catheter (27) for delivery of medications within the intramedullary canal of the long bone is facilitated.

## Patentansprüche

1. In Kombination:
eine Zielführung (10), die einen Griffabschnitt (34) und einen Schaftabschnitt (42) umfasst, wobei der Schaftabschnitt (42) eine Bohrung (52) aufweist, die im Wesentlichen entlang seiner Längsachse (54) verläuft, und wenigstens einen ersten Kanal (50); und
einen Marknagel (12), der am Schaftabschnitt (42) befestigt ist, wobei der Marknagel (12) wenigstens einen zweiten Kanal (26) aufweist, der am ersten Kanal (50) ausgerichtet ist, wobei die Kanäle (26, 50) dazu konfiguriert sind, einen Katheter (27) aufzunehmen.

2. Kombination von Anspruch 1, wobei der Schaftabschnitt (42) ein Paar erster Kanäle (50) aufweist und der Marknagel (12) ein Paar zweiter Kanäle (26) aufweist, wobei die ersten (50) und die zweiten (26) Kanäle jeweils aneinander ausgerichtet sind.

3. Kombination nach Anspruch 2, wobei wenigstens einer der ersten (50) und/oder der zweiten (26) Kanäle eine Innenfläche (29, 51) aufweist, die sich durch einen Winkel von wenigstens 180° erstreckt.

4. Kombination nach Anspruch 1, wobei der erste (50) und der zweite (26) Kanal entlang einer gemeinsamen ersten Achse ausgerichtet sind.

5. Kombination nach Anspruch 1, wobei der Schaftabschnitt (42) und der Marknagel (12) entlang einer gemeinsamen zweiten Achse ausgerichtet sind, wobei die zweite Achse parallel zur ersten Achse verläuft.

6. Kombination nach Anspruch 1, wobei der Schaftabschnitt (42) ferner Mittel zum festen Eingreifen des Marknagels (12) umfasst.

7. Kombination nach Anspruch 1, wobei der Katheter (27) durch die ausgerichteten Kanäle (26, 50) eingeführt werden kann.

8. Kombination nach Anspruch 1, wobei die Kombination ferner einen Katheter (27) umfasst.

9. Kombination nach Anspruch 8, wobei der Katheter (27) vor dem Einsetzen des Marknagels (12) in einen Markraum eines Röhrenknochens im Kanal (26) des Schaftabschnitts (42) und im Kanal (50) im Marknagel (12) aufgenommen wird, so dass das Einbringen des Katheters (27) zur Verabreichung von Medikamenten innerhalb des Markraums des Röhrenknochens ermöglicht wird.

## Revendications

1. En combinaison :
un guide de ciblage (10) incluant une partie poignée (34) et une partie cylindre (42), ladite partie poignée (42) incluant un alésage (52) s'étendant sensiblement le long de son axe longitudinal (54) et au moins un premier canal (50) ; et
un clou intramédullaire (12) solidarisé à ladite partie cylindre (42), ledit clou intramédullaire (12) incluant au moins un second canal (26) aligné avec ledit premier canal (50), lesdits canaux (26, 50) étant configurés pour recevoir un cathéter (27).

2. Combinaison selon la revendication 1, dans laquelle ladite partie cylindre (42) inclut une paire desdits premiers canaux (50) et ledit clou intramédullaire (12) inclut une paire desdits seconds canaux (26), lesdits premiers (50) et seconds (26) canaux étant respectivement alignés les uns avec les autres.

3. Combinaison selon la revendication 2, dans laquelle au moins l'un desdits premiers (50) et seconds (26) canaux inclut une surface interne (29, 51) qui s'étend sur un angle d'au moins 180°.

4. Combinaison selon la revendication 1, dans laquelle lesdits premiers (50) et seconds (26) canaux sont alignés le long d'un premier axe commun.

5. Combinaison selon la revendication 1, dans laquelle ladite partie cylindre (42) et ledit clou intramédullaire (12) sont alignés le long d'un second axe commun, ledit second axe étant parallèle audit premier axe.

6. Combinaison selon la revendication 1, dans laquelle ladite partie cylindre (42) comprend en outre un moyen permettant de mettre en prise de façon fixe ledit clou intramédullaire (12).

7. Combinaison selon la revendication 1, dans laquelle le cathéter (27) peut être inséré à travers les canaux alignés (26, 50).

8. Combinaison selon la revendication 1, dans laquelle la combinaison comprend en outre un cathéter (27).

9. Combinaison selon la revendication 8, dans laquelle le cathéter (27) est reçu dans le canal (26) de la partie cylindre (42) et le canal (50) dans le clou intramédullaire (12) avant l'insertion du clou intramédullaire (12) dans un canal intramédullaire d'un os long de sorte que le placement du cathéter (27) destiné à l'administration de médications au sein du canal intramédullaire de l'os long soit facilité.
